# EUROPEAN PATENT APPLICATION

(11) **EP 4 670 747 A2**
(43) Date of publication of application: **31.12.2025**
(21) Application number: 25216738.2
(22) Date of filing: 15.03.2022
(51) Int. Cl.: A61L 17/12

(54) **SUTURES**

(30) Priority: 15.03.2021 GB 202103567
(62) Divisional of application: 22715055.4
(71) Applicant: The Queen's University of Belfast, Belfast BT7 1NN (GB)
(72) Inventor: LI, Shu, Belfast, BT7 1NN (GB); ANDREWS, Gavin P., Belfast, BT7 1NN (GB); JONES, David S., Belfast, BT7 1NN (GB)
(74) Representative: FRKelly

(57) **Abstract**

The invention relates to a suture thread material comprising at least one polymer and at least one eutectic mixture.

The polymer can be a biocompatible polymer such as polycaprolactone or PLGA.

The eutectic mixture comprises triclosan.

Other eutectic mixtures can be used in suture thread materials to reduce SSis and/or to decrease pain and/or to increase lubricity of sutures.

The invention also provides a method for the production of sutures with drug loadings, the method comprising the use of therapeutic deep eutectic solvent (THEDES) technology to significantly increase drug content in suture matrices.

## Description

### Field of the Invention

The present invention relates to suture threads. More particularly the invention relates to anti-infective sutures using deep eutectic reactive extrusion technology

### Background to the Invention

Sutures play a prominent role as wound closure devices, with an estimated market value of $3.68 billion in 2017, and projections valuing growth to $5.02 billion by 2023. A recognised disadvantage of sutures, however, is that they can, like most implanted medical devices, be susceptible to bacterial adherence and colonization, which eventually leads to development of surgical site infection (SSIs) as bacteria permeates into the wound bed. This results in impeded wound repair due to wound dehiscence and deep wound tissue abscess. Infection of the wound subsequently triggers an elevated immune response, causing inflammation and tissue damage in close proximity to the suture site, retarding healing. Extended wound healing utilises already limited hospital resources, by increasing number of hospital readmissions, outpatient visits, as well as increased overall costs of wound care/management as a result of the need for antimicrobial dressings or alternative course of antimicrobial and/or anti-inflammatory treatments.

Surgical site infections (SSIs) cause severe problems and remain a major cause of postoperative mortality, particularly at incision sites that are considered 'dirty-contaminated' according to the CDC Surgical Wound Classification (SWC Class IV). The incidence of SSIs at Class IV wound sites has been reported to be over 27% in general and can be as high as 40% in extreme cases such as in abdominal laparotomy and intestinal procedures, particularly colostomies. Occurrence of SSIs may result in repeat surgeries, use of systemic antibiotics and, prolonged hospital stays with approximate additional increased healthcare costs of up to $60,000 per patient.

Amongst a number of causative aetiologies for unhealed wounds, infection and subsequent inflammation at the surgical incisional site is regarded as the most important and preventable cause. A short course of antimicrobial agent before a surgical procedure, known as antimicrobial prophylaxis, is a common technique in order to reduce the likelihood of developing an SSI. As oral delivery is one of the most widespread routes of administration of these antimicrobials, the medication is usually given to the patient in this fashion, frequently resulting in high doses due to low oral bioavailability, as well as unpleasant side effects due to high systemic levels of the drug. In order to minimise this, an antimicrobial drug or combination of drugs with varying properties, can be delivered locally to the site of surgery through embedment of the chosen drug(s) into wound care devices such as wound dressings, sutures, etc.

When surgical procedures were performed to deep tissues or internal organs, such as the colorectal regions and appendectomy surgeries, drug delivery becomes increasingly difficult. For these deep tissue surgeries, the development of an SSI may have serious consequences, due to the site being physically difficult to access. These deep tissue SSIs can have a high risk of mortality to the patient, with patients undergoing coronary artery bypass surgery found to have a mortality rate of 22 % if a deep SSI was developed, compared to a mortality rate of 0.6 % with no infection. As well as the high rate of mortality, there is a huge economic impact with these patients, with patients developing SSIs requiring, on average, an additional 20 days in hospital, with patients who died as a result of deep SSIs costing on average over $60,000 more than patients who survived (11). As a result, incorporating one or more active agents (APIs) such as compounds with antimicrobial activity into the suture material becomes increasingly important. The delivery of drugs including antimicrobials and/or antibiotics directly to the site of the wound through incorporation of an API into the suture, provides a highly efficient method for localised drug delivery. Through the use of sutures which possess antimicrobial properties, the risk of SSIs is therefore reduced. This results in a high local concentration of the drug at the wound site, while eliminating unpleasant side-effects resulting from high systemic levels of the drug throughout the whole body.

Since the approval (2002) of the first antimicrobial-coated surgical suture (VICRYL^{®} Plus Antibacterial by Ethicon Inc.), functional sutures have emerged as a promising means of assisting with the prevention of SSIs. The Royal College of Surgeons of England supports the use of antimicrobial sutures (21st July 2016), particularly in surgical procedures leading to SW Class II-IV wounds (Surgical Wound Classification) where SSI rate is high (i.e. colorectal surgery, C-section, gastric surgery, liver/kidney transplant, gun-shot wound to the abdomen) or when an SSI can be life-threatening even with low incidence rates (i.e. cardiac surgery). The WHO Implementation Manual to Support the Prevention of SSIs at the Facility Level (2018) recommends the use of triclosan-coated sutures to reduce incidence of intraoperative infections. The NICE Guidelines also recommends (2019) that "when using sutures, they should be antimicrobial triclosan-coated, especially when undertaking paediatric surgery, to reduce the risk of surgical site infection".

To date, a number of studies have demonstrated methods to include drugs either onto the surface of existing sutures through surface modifications such as coating or grafting, onto the suture threads made via melt or electro-spinning, or impregnated into the matrix of preformed sutures by soaking in drug containing solvents or other advanced liquids (supercritical CO₂). The majority of these established methods require the use of solvents and are, therefore, subject to solvent retention. Moreover, the need to remove solvent inevitably introduces drying of the products hence resulting in multiple stepped and labour-intensive processes. More importantly, one particular disadvantage of these methods is the limited drug loading (typically <20% w/w) that can be achieved without detrimentally impacting suture characteristics (such as the mechanical strength, knot security, coefficient of friction). Such limited drug content, coupled with the unique miniatured nature of this type of wound-closing device then renders the drug-loaded sutures less effective than intended, particularly in severe cases of SSIs. Indeed, the existing antiseptic-coated sutures merely prevent biofilm formation on the surface of the suturing threads, rather than releasing the active agent to ensure a bacteria-free zone surrounding the stitched surgical site. In addition, insufficient antimicrobial at the site of infection may proliferate genetic mutation of the bacteria, subsequently leading to rapid development of antimicrobial resistance (AMR).

The recent development of drug-eluting sutures presents a potential solution to offer localised drug delivery for improved wound healing. Despite this, there are significant limitations associated with achieving optimal drug loading without negatively impacting the mechanical properties of the threads. Such limited drug loading has posed challenges to utilise antimicrobial sutures as effective drug delivery devices.

Compared to monotherapies, synergistic combination therapies where two or more antimicrobial agents are used concurrently have been reported to prevent AMR and to offer superior outcomes against multiple-drug resistant (MDR) pathogens typically via providing different complementary mechanisms. The concept of combination remedies for anti-infective treatment, albeit remaining controversial, may be advantageous when resistance to a single agent develops rapidly, or when the combination brings significantly improved antimicrobial efficacy when compared with either parent agent.

### Brief description of the Invention

This invention relates to the use of eutectic mixtures in polymer based suture threads.

The invention provides a suture thread comprising at least one polymer and at least one eutectic mixture.

The eutectic mixture may comprise one or more agents and one or more of the group selected from a fatty acid, a fatty amine, a fatty alcohol, or any combination of two or more thereof.

The present invention may provide a suture thread comprising:
a) at least one biocompatible polymer; and
b) an eutectic mixture containing at least one active pharmaceutical ingredient.

The eutectic mixture may comprise a first agent and one or more of the group comprising
i) one or more active agents capable of forming a eutectic composition with the first agent; or
ii) one or more fatty acids; or
iii) one or more fatty alcohols; or
iv) one or more fatty amines; or
v) any combination of i) to iv) above, including any combination of two or more of i) to iv).

Preferably the polymer is a biodegradable biocompatible polymer.

A preferred polymer is or includes polycaprolactone.

In a preferred embodiment the biocompatible polymer is biodegradeable. The polymer may be capable of accommodating *in situ* formation, during extrusion processing, of the eutectic mixture in or on the polymer.

Biodegradable polymers may be preferred when taking into consideration potential resilience, from regulatory and practical perspectives, to apply antibiotic-loaded sutures to surgical sites. The sutures, such as anti-infective sutures, may need to be applied to surgical sites that are more prone to SSIs yet more difficult to manage with alternative non-systemic treatments. Those sites would include deep tissues and organ space, where after the surgery would be impossible to access unless the wound bed is opened up again.

The biodegradation profile of polycaprolactone was a major reason for its use in the preliminary work. Other reasons included cost, availability in the labs. PLGA is another preferred biodegradable biocompatible polymer.

In an alternative embodiment, the biocompatible polymer is substantially non-biodegradeable. The polymer may be capable of accommodating *in situ* formation, during extrusion processing, of the eutectic mixture in or on the polymer.

In various embodiments, the at least one biocompatible polymer is selected from the group comprising poly ethylene-vinyl-acetate (EVA), vinyl acetate, silicone, polycaprolactone , polyglycolic acid (PGA), polylactic co-glycolic acid (PLGA), polylactic acid (PLA), polydioxanone, poliglecaprone (copolymer of glycolide and epsilon-caprolactone), poly-(trimethylene carbonate)-based polymers, polypropylene (nonbiodegradable), polyester (nonbiodegradable), nylon (nonbiodegradable). In specific examples, the polymer is polycaprolactone.

In a preferred embodiment, one of the eutectic forming components is maleic acid.

In a preferred embodiment, one of the eutectic forming components is comprises metronidazole.

In a preferred embodiment, two of the eutectic forming components aremaleic acid and metronidazole.

In certain embodiments, the eutectic mixture comprises at least one fatty acid, fatty alcohol and/or fatty amine to generate liquid eutectic system in situ within a polymer device.

One of the eutectic forming components may comprise at least one fatty acid.

The fatty acid may be a mono or dicarboxylic fatty acid comprising 4 to 20 carbon atoms (optionally 4 to 18 carbon atoms) that is saturated or unsaturated, or mixtures thereof. The fatty acid may be selected from maleic acid (C4; unsaturated), hexanoic acid (C6), hexanedioic acid (C6), capric acid

(C10), decanedioic acid (C10), lauric acid (C12), dodecanedioic acid (C12; unsaturated), tetradecanedioic acid (C14; unsaturated), myristic acid (C14) or Oleic acid (C18).

Mixtures of fatty acids are envisaged, including mixtures of capric acid and lauric acid ; mixtures of capric acid and myristic acid ; mixtures of oleic acid and lauric acid ; and mixtures of oleic acid and capric acid.

The eutectic mixture may comprise a fatty acid and a second fatty acid, a fatty alcohol, a fatty amine or a drug or other agent.

The invention provides the use of anti-infective liquid eutectic mixtures to reduce surgical site infections (SSIs).

The invention provides the use of eutectic mixtures to increase the lubricity of sutures.

For sutures made of materials of high coefficient of friction (for example polyglactin - a copolymer made from 90% glycolide and 10% L-lactide), certain level of additional lubrication could help reduce tissue-dragging and avoid microtrauma during application.

The suture thread generates eutectic oils in situ within sutures which exude from the material and in so doing can render the surface lubricious.

The invention provides the use of anti-infective liquid eutectic mixtures to reduce surgical site infections (SSIs) and to increase the lubricity of sutures.

The eutectic mixture can comprise a drug (active agent) capable of forming a eutectic mixture with a fatty acid, alcohol or amine.

The fatty alcohol may be a straight-chain or branched-chain, saturated or unsaturated primary alcohol, ranging from as few as 4-6 carbons to as many as 26-30 carbons. The fatty alcohol may be 3-methyl-3-pentanol (C6); 1-Dodecanol (C12), Heneicosanol (C21), Elaidyl alcohol (C18; monounsaturated), Petroselinyl alcohol (C18; monounsaturated), 1-tetradecanol (C14), 1-Docosanol (C22; saturated), 1-Tridecanol (C13), 1-Nonadecanol (C19), 1-Triacontanol (C30), 1-Pentadecanol (C15), 1-Nonanol (C9), 1-Tricosanol (C23), Cetyl alcohol (C16), Stearyl alcohol (C18), 1-Decanol (C10), or Isooctyl alcohol (C8).

The fatty amine may be a straight-chain or branched-chain, saturated or unsaturated amine that is, optionally, mono- or di-substituted with a short chain alkyl (optionally, methyl) at the amino group. The fatty amine may have as few as 4-6 carbons to as many as 22-26 carbons. The fatty amine may be Pentadecylamine (C15), Hexadecylamine (C16), Docecylamine (C14), Decylamine (C10), Tridecylamine (C13), Octadecylamine (C18), Undecylamine (C11), N, N-dimethyltetradecylamine (C16), Dodecylamine (C12), or Octadecylamine (C18).

In one embodiment the drug (active agent) is metronidazole.

In embodiments the drug may be
(1) an antibacterial with the nitroimidazole scaffold, such as metronidazole, tinidazole, nimorazole;
(2) an antifungal having the imidazole scaffold including ketoconazole, econazole, clotrimazole, tioconazole;
(3) an antiviral that is a derivative of either imidazole or nitroimidazole;
(4) an anti-inflammatory that consists of the carboxylic acid functionality, such as derivatives of propionic acid (ibuprofen, naproxen), derivatives of acetic acid (indomethacin, diclofenac), derivatives of anthranilic acid (mefenamic acid, meclofenamic acid); or
(5) a local anesthetic comprising functional groups with strong group electronegativity such as an amide (lidocaine, prilocaine, bupivacaine, etc.), an ester (procaine, benzocaine, tetracaine, etc.).

By using a eutectic form of a chosen anti-microbial with coforming agents showing synergistic anti-microbial activities either via additive effects or different mechanisms of action, the anti-infective capability is enhanced.

By using a eutectic form of a drug within a polymer carrier, the drug loading and delivery capacity is increased.

Having the drug present in the polymer carrier in a eutectic form allows for higher drug loading with less negative impact upon the mechanical properties of the suture threads.

The controlled delivery of drugs through sutures can improve the management of post-operative pain, inflammation and infection, promoting faster post-operative recovery.

The controlled delivery of local anaesthetic or analgesia through sutures can decrease opiate consumption.

The localized and controlled delivery of antimicrobial (be it an antibiotic, a non-antibiotic antimicrobial, or substances with natural antimicrobial activities) can reduce the needs for additional post-surgical anti-infective treatment such as antimicrobial wound dressings and/or systemic (usually orally administered) antibiotic courses.

In one embodiment, the invention provides a suture thread loaded with an antibiotic and a fatty acid in a eutectic mixture.

In one particular embodiment, the invention provides a suture thread loaded with metronidazole in a eutectic 1:1 mixture with maleic acid, in a polycaprolactone manufactured using hot melt extrusion.

Other particular combinations include the following:
- Metronidazole-Fatty Acids
- Metronidazole - hexanoic acid (C6); metronidazole - hexanedioic acid (C6): NOTE: A dicarboxylic fatty acid.
- Metronidazole - Non-steroidal anti-inflammatory (NSAIDs)
- Metronidazole - Ibuprofen; metronidazole - Naproxen.
- Triclosan (with functional groups representative of both ethers and phenols)- Fatty Acids
- Triclosan - Maleic acid (C4: A dicarboxylic fatty acid).
- Triclosan - NSAIDs
- Triclosan - Ibuprofen
- Lidocaine - NSAIDs
- Lidocaine - Ibuprofen; lidocaine - naproxen
- Lidocaine - fatty acids
- Lidocaine - capric acid (C10); Lidocaine - decanedioic acid (C10); Lidocaine - lauric acid (C12); Lidocaine - dodecanedioic acid (C12; A dicarboxylic fatty acid:
- Lidocaine - tetradecanedioic acid (C14: A dicarboxylic fatty acid:
- Lidocaine-Fatty Alcohols
- Lidocaine - 3-methyl-3-pentanol (C6); Lidocaine - 1-decanol (C10); Lidocaine - 1-dodecanol (C12); Lidocaine - 1-tetradecanol (C14).
- Prilocaine-Fatty Acids
- Prilocaine - hexanedioic acid (C6): A dicarboxylic fatty acid; Prilocaine - decanedioic acid (C6): NOTE: A dicarboxylic fatty acid; Prilocaine - dodecanoic acid (C12, Lauric Acid)
- Ropivacaine-Fatty Acids
- Ropivacaine - decanoic acid (C10); Ropivacaine - decanedioic acid (C10): NOTE: A dicarboxylic fatty acid.
- Ropivacaine - dodecanoic acid (C12).
- Ropivacaine-Fatty Alcohols
- Ropivacaine - 1-decanol (C10); Ropivacaine - 1-tetradecanol (C14)
- Fatty Acids-Fatty Acids
- Capric acid (65% w/w) and Lauric acid (35% w/w); Capric acid (75% w/w) and Myristic acid (25% w/w); Oleic acid (80% w/w) and Lauric acid (20% w/w); Oleic acid (80% w/w) and Capric acid (20% w/w).

The following Table provides summarised eutectic forming binary components, along with their respective eutectic forming composition ranges:

**Table 1**

| | **Fatty acids** | **Fatty alcohols** | **NSAIDs** |
|---|---|---|---|
| **Antimicrobials** *(including antibiotics, non-antibiotic antimicrobials, antivirals, antifungals and antiparasitics) that are* a *derivative of the imidazole scaffold* | **Metronidazole** - **Maleic acid** (1:1 ideal and complete reaction with liquid portions visible and measurable from 1:9 - 4:1)* | | **Metronidazole - Ibuprofen** (1:1 ideal and complete reaction) |
| | | | **Triclosan** - **Ibuprofen** (1:1 complete reaction with liquid portions measurable across entire composition range) |
| **Local anaesthetics** *possessing amide groups* | **Lidocaine - Capric acid** (1:4 - 3:2) | **Lidocaine** - **1-Decanol** (=< 1:2) | **Lidocaine** - **Ibuprofen** (3:7-7:3) |
| | **Lidocaine - Hexanoic acid** (1:6 - 4:1) | **Lidocaine** - **1-dodecanol** (1:1) | |
| | | | **Lidocaine** - **Ketoprofen** (1:1) |
| | **Lidocaine - Lauric acid** (1:1) | **Lidocaine** - **1-tetradecanol** (1:1) | |
| | **Lidocaine - Adipic acid** (1:5 - 3:1) | **Lidocaine** - **3-Methyl-3-Pentanol** (1:1) | |
| | **Lidocaine - Sebacic acid** (2:3 - 3:1) | | |
| | **Lidocaine - Myristic acid** (1:1) | | |
| | **Lidocaine** - **Dodecanedioic acid** (1:1 - 3:1) | | |
| | **Lidocaine - Tetradecanedioic acid** (1:1) | | |

| | | | |
|---|---|---|---|
| ***Note the compositions are written as molar ratios in this table.** | | | |

Features of certain embodiments of the suture include any one or more of the following:
- Drug content is uniformly spread along the length of the body of the polymeric matrices. This allows for the suture to be cut as required.
- The device is in some embodiments made of a biodegradable or non-biodegradable polymer, or a mixture of biodegradable and non-biodegradable polymers.
- Suture threads with different layers, including different actives and/or having different release profiles and could be used to allow differential release profiles.
- The present invention may provide a suture that acts as a bioactive drug delivery platform for improved post-surgical wound care and management wherein the suture comprises therapeutic deep eutectic solvent (THEDES) technology to increase drug content in sutures.

The invention also provides a method for the production of sutures with high antimicrobial combination loadings, the method comprising the use of therapeutic deep eutectic solvent (THEDES) technology to significantly increase drug content in suture matrices.

THEDES allows for a liquid to be formed at room temperature between a drug and a counter-ion ingredient, or between two drugs that show differences in primary pKa values, through noncovalent forces such as H-bonding, or charge-assisted H-bonding.

Data illustrates superior antimicrobial activity relative to either parent agent.

The invention thus provides suture thread for use as a bioactive drug delivery platform for improved post-surgical wound care and management.

The invention describes the use of drug combinations engineered into the matrix of sutures at elevated loadings in an attempt to address both SSIs and AMR issues.

Active agents including antiseptics (e.g., triclosan, chlorhexidine), antimicrobial lipids (such as short- or medium-chain fatty acids) and/or choline-based deep eutectic solvents (DES) will be incorporated into suture threads at high-loading using reactive hot-melt extrusion (RHME), which is a solvent-less, continuous and industrially scalable technique.

The inventors have successfully proven viability of in-house production of drug-loaded thin filaments with a diameter ranging between 100-400 micron (equivalent to USP suture sizes 5-0 to 1).

More importantly, it has proven feasible to incorporate substantially elevated drug loadings (>30% w/w) without significantly compromising the physical and mechanical characteristics of the extruded threads using THEDES technology.

Moreover where higher loadings are required, co-axial multi-layer extrusion is used to produce core-sheath layered suture threads, that offer the ambidexterity of extreme drug loading and excellent mechanical strength.

### Detailed Description of the Invention.

### Brief description of the figures.

The invention is described in greater detail with reference to the following figures wherein
**Figure 1** shows overlaid FT-IR spectra in the regions of 1800 - 1500 cm⁻¹ and 3600 - 2400 cm⁻¹ for MET-MA 1:1, (MET-MA 1:1)-PCL physical mixture and extrudate (containing 10 % and 20 % w/w MET) and pure PCL. From top to bottom, the traces are PCL, 20% extrudate, 20% mix, 10% extrudate, 10% mix and MET-MA 1:1. Ratios are molar unless otherwise stated.
**Figure 2** shows overlaid FT-IR spectra in the regions of 1800 - 1500 cm⁻¹ and 3600 - 2400 cm⁻¹ for MET-MA 2:1, (MET-MA 2:1)-PCL physical mixture and extrudate (containing 40 % and 50 % w/w MET) and pure PCL.. From top to bottom, the traces are PCL, 50% extrudate, 50% mix, 40% extrudate, 40% mix and MET-MA 2:1.
**Figure 3** is a picture of twin screw Rondol Microlab bench-top extruder used in this work.
**Figure 4** is a picture of screw elements used here; (a) full conveying (FC) (b) 90 ° and (c) 60 ° mixing element (MIX) geometries.
**Figure 5** shows content uniformity, represented by % recovery, of MET-MA (2:1)-PCL 40 % w/w MET formulation after extrusion using the HAAKE Minilab (non-intermeshing screws) and the Rondol Microlab extruder (FC and MIX elements).
**Figure 6** shows zone of Inhibition (ZOI) testing using disc diffusion method to confirm antimicrobial efficacy of the chosen antibiotic agent and SCFA, both as single agent and as a dual-combination (THEDES), against pre-grown *Pseudomonas aeruginosa* on agar plates. The tests were compared to a negative (PBS) and a positive (gentamicin) control, respectively.
**Figure 7** shows representative inhibition zone assay on *Staphylococcus aureus* NCTC 10788 cultures using blank membrane disks loaded with MET and MA (6 mg/mL) in solutions of PBS, neat MET-MA THEDES, and gentamicin sulphate (10 mg/mL) and PBS as positive and negative controls, respectively.
**Figure 8** shows representative inhibition zone assay on *Klebsiella pneumoniae* ATCC 700603 cultures using blank membrane disks loaded with MET and MA (6 mg/mL) in solutions of PBS, neat MET-MA THEDES, and gentamicin sulphate (10 mg/mL) and PBS as positive and negative controls, respectively.
**Figure 9** shows representative plate sections showing the ZOIs against *Pseudomonas aeruginosa* formed by blank PCL, Vicryl^{®} Plus, MET-PCL at 10 % w/w and 20 % w/w MET, (MET-MA 1:1)-PCL at 10 % w/w and 20 % w/w MET and (MET-MA 2:1)-PCL at 40 % w/w and 50 % w/w MET.
**Figure 10** shows representative plate sections showing the ZOls against *Klebsiella pneumoniae* formed by blank PCL, Vicryl^{®} Plus, MET-PCL at 10 % w/w and 20 % w/w MET, (MET-MA 1:1)-PCL at 10 % w/w and 20 % w/w MET and (MET-MA 2:1)-PCL at 40 % w/w and 50 % w/w MET.
**Figure 11** shows tensile failure forces (N) of sutures (USP 3-0) produced through HME, loaded with MET or MET-MA THEDES. Data shown is the mean ± S.D., where n = 3.
**Figure 12** shows dissolution profiles of melt extruded MET, MET-MA 1:1 and MET-MA 2:1 formulations with PCL in PBS pH 7.4 and 37 ± 0.5 °C. Each point is representative of the mean ± S.D., where n = 3. MET 10% and MET 20% are grouped and are all below about 40% % cumulative MET release.
**Figure 13** shows a temperature-molar ratio phase diagram of Metronidazole-Maleic acid binary system, with liquidus shown as open circles and Tg of the binary mixtures shown as filled circles. Each plot is the average of three replicates (mean ± S.D.).
**Figure 14** shows DSC phase diagrams for (a) Lidocaine - Hexanoic acid, (b) Lidocaine - Capric acid, (c) Lidocaine - Lauric acid and (d) Lidocaine - Myristic acid binary mixtures at varying compositions with liquidus shown as filled squares and Tg of the binary mixtures shown as filled circles.
**Figure 15** shows DSC phase diagrams for (a) Lidocaine - Adipic acid (hexanedioic acid), (b) Lidocaine - Sebacic acid (1,8-Octanedicarboxylic acid), (c) Lidocaine - Dodecanedioic acid and (d) Lidocaine - Tetradecanedioic acid binary mixtures at varying compositions. Liquidus is shown as filled squares and Tg of the binary mixtures is shown as filled circles.
**Figure 16** shows DSC phase diagrams for (a) Lidocaine - 3MetPen (3-methyl-3-pentanol), (b) Lidocaine - 1-decanol, (c) Lidocaine - 1-dodecanol and (d) Lidocaine - 1-tetradecanol binary mixtures at varying compositions. Liquidus is shown as filled diamonds and Tg of the binary mixtures is shown as filled squares.
**Figure 17** shows DSC thermogram of triclosan (TRC), ibuprofen (IBU) and an equimolar (1:1) physical mixture of TRC-IBU in the region of -65-50 °C on the second heat curve of a heat-cool-heat cycle. The top trace is TRC; the middle trace is IBU and the bottom trace is TRC-IBU. The equimolar binary mixture formed a miscible system characterised by a single glass transition (Tg) on the second heat curve between the individual glass transition events of the two parent components. This formed equimolar mixture was observed to be a clear RT liquid.
**Figure 18** shows DSC thermograms of (from top to bottom) triclosan and ibuprofen, TRC-IBU _{X}TRC = 0.9, 0.8, 0.7, 0.6, 0.5, 0.4, 0.3, 0.2 and 0.1, physical mixtures *(right hand drawing) and* after heating at 10 °C/min to 200 °C *(left hand drawing).*
**Figure 19** shows a phase diagram of the TRC-IBU system, where the red dots form a shallow "V"-shape and represent the liquidus, the black dots form a rectilinear line and represent the solidus and the blue dots (at the bottom) also form a rectilinear line and show the glass transition of the coamorphous system. The grey line (with no dots) represents the theoretical liquidus lines, calculated using the Schröder-Van Laar equation. The grey line is broadly consistent with the empirically determine liquidus.
**Figure 20** shows temperature-molar ratio phase diagram of lidocaine-ibuprofen (LID-IBU) binary system, each plot is the average of three replicates (mean ± S.D.).
**Figure 21** *shows (left)* LID-IBU equimolar mixture extruded, without polymer, at 60°C showing clear RT liquid (deep) eutectic formation. *(right)* extruded LID-IBU equimolar THEDES exhibiting characteristic charge-assisted H-bonding between the carboxylic acid group from IBU and the amide group from LID.
**Figure 22** shows equimolar lidocaine and ketoprofen. THEDES formation is indicated by DSC (complete loss of melting events) and FTIR (characteristic charge-assisted H-bonding pattern between LID amide and ketoprofen carboxylic acid). In the left-hand drawing, the traces are, from the top, lidocaine, Keto:Lido (1:1)THEDES and Ketoprofen,. In the right hand drawing, the traces are, from the top, ketoprogen, Keto_Lido (1:1) THEDES and lidocaine.

### Definitions

Surgical sutures are a type of wound closing medical device that facilitates healing of the open wounds by holding body tissues together. Dependent upon the material used, sutures can be broadly categorised into non-absorbable and absorbable sutures. For a variety of application requirements (such as tolerance to tissue drag, flexibility, knot security, tensile strength, size) sutures can be produced as single-threaded monofilament, or multifilament with braided threads.

The terms "surgical suture", "suture" and "suture thread", as used herein, are interchangeable.

A suture can be a single thread, or monofilament, or a suture can be multiple threads, or multilfilaments wherein the filaments are braided together. The term "suture thread", as used herein, is intended to include both single threads and multiple threads.

The term "eutectic" was coined in 1884 to describe binary (or multiple component) systems that have "a lower temperature of liquefaction than that given by any other proportion", i.e. melting at a lower temperature compared to the individual components. Each component of the eutectic system is termed, herein, a eutectic forming component.

Deep eutectics (DESs) fall within the definition of "eutectic" above but usually consist of a Lewis or Brønsted-Lowry acid and base pair (such as a carboxylic acid/choline bicarbonate pair, a carboxylic acid/nitroimidazole pair, a carboxylic acid/amide pair), demonstrating strong interaction. Natural deep eutectics (NADES) are bio-based and are composed of two or more eutectic forming components, i.e. organic acids, sugars, alcohols, amines and amino acids. Deep eutectics are characterized by a profound depression in melting (liquefaction) points, hence the term *deep.* Accordingly, the resulting system is usually a viscous liquid with low volatility at room temperature.

When at least one of the forming components in a DES is an active pharmaceutical ingredient (API), the DES is termed a therapeutic DES (THEDES).

As used herein, the liquidus lines on a phase diagram is the locus of all system states that represent the boundary between a single liquid phase and the two phase (liquid + solid) zones on the diagram.

### Methods: Preparation of THEDES loaded sutures via In-Situ Reactive Extrusion

The hot melt extrusion method was established in the early 1930s. Extrusion is used to change the physical properties of the raw materials by pushing them through a die of the desired cross section under elevated controlled temperature and pressure. Hot melt extrusion involves multiple compaction steps and conversion of the powdered ingredients into a product of uniform density and shape. The rotating screw(s) force the polymer and eutectic forming components forward toward the die under controlled temperature, pressure, feeding rate, and screw speed.

The extruder may consist of single or twin rotating screws (co-rotating or counter-rotating) inside a stationary cylindrical barrel. A plate die is connected to the end of the cylindrical barrel designed based on the desired shape of the extruded material.

Single-screw extruders are widely used hot melt extruders because they are mechanically simple devices. A single-screw extruder consists of one rotating screw positioned inside a stationary barrel that results in good-quality molten material and generates a high stable pressure for a consistent output. In general, single-screw extruders include a feed zone, a compression zone, and a metering zone. The single-screw extruder receives the raw material in the feeding zone with very low pressure by increasing the screw pitch and/or the screw flight depth, larger than that of other zones in order to allow for consistent feeding from the hopper and gentle mixing of the polymer and eutectic forming components. In the compression zone, the pressure is increased by decreasing the screw pitch and/or the flight depth to effectively impart a high degree of mixing and compression of the polymer and he eutectic forming components. Finally, in the metering zone, the molten extrudate is pumped through a die that imparts a definite shape for further downstream processing including cooling, cutting, and collecting the finished product.

Twin-screw extruders consist of two closely matched screws inside the extruder barrel. The use of two screws permits different types of configurations and imposes different conditions in all the extruder zones, from the feed zone to the rotating screw in the compression zone, and finally to convey the material to the metering zone. The rotation of the screws in twin screws may either be co-rotating (same direction) or counter-rotating (opposite direction). The two types of twin-screw extruders can be further classified into: (1) fully intermeshing and (2) non-intermeshing. The fully intermeshing type is more frequently used due to the self-cleaning feature and reduces non-motion by preventing localized overheating of raw materials with the extruder. The non-intermeshing type is less popular in the mixing application due to its weaker screw interactions and lower self-cleaning capability. Both types are often used to process highly viscous materials. However, the non-intermeshing type is not susceptible to high torque generation while processing highly viscous materials because these screws are positioned separately from each other
The following data were generated using this method.

Reactive extrusion was performed using a twin-screw intermeshing co-rotating compounder equipped with pre-configured screw elements for sufficient conveying/kneading/discharging, and a filament die of the desired diameter; for example a 2 mm diameter filament die. Ternary physical mixtures containing the individual eutectic forming components, and the polymer were fed to the extruder and processed at a fixed screw rotation speed of 30 rpm and extruder zone temperatures appropriate for the respective polymer (detailed in Table 1 for PCL as an example polymeric carrier). Eutectic formation occurred *in-situ* within the extruder barrel with the formed THEDES immediately dispersed throughout the molten polymeric matrix. Upon exiting through the die, the extruded filaments were pulled at various rates prior to air-cooling in order to produce a variety of sizes of suture threads.

The presented DES-embedded sutures in the figures were processed using an intermeshing extruder. We have also tried non-intermeshing extrusion. The tried DES could form during non-intermeshing extrusion. However, the recovered active content from those extrudates, during content uniformity assessment ,was a little poor (although improvements were seen with prolonged processing time).

**Table 2 Reactive extrusion processing parameters for in-situ eutectic forming suture preparation using PCL as an example polymeric carrier.**

| **Zone Zero (Feeding)** | **Zone One** | **Zone Two** | **Zone Three** | **Die (Exiting)** |
|---|---|---|---|---|
| **Temperature setting (when PCL was used)** | | | | |
| **55 °C** | 65 °C | 65 °C | 65 °C | 60 °C |

| **Screw configuration** | | | | |
|---|---|---|---|---|
| **5 x Conveying** | 1 x Conveying | 1 x 60° Kneading | 1 x 90° Kneading | N/A |
| | 2 x 60° Kneading | 2 x Conveying | | |
| | 2 x 90° Kneading | 2 x 60° Kneading | 1 x 60° Kneading | |
| | | | | |
| | | | 3 x Conveying | |
| The following Table will provide summarised eutectic forming binary components, along with their respective eutectic forming composition ranges: | | | | |

**Table 3**

| | **Fatty acids** | **Fatty alcohols** | **NSAIDs** | |
|---|---|---|---|---|
| **Antimicrobials** *(including antibiotics, non-antibiotic antimicrobials, antivirals, antifungals and antiparasitics) that are* a *derivative of the imidazole scaffold* | **Metronidazole** - **Maleic acid** (1:1 ideal and complete reaction with liquid portions visible and measurable from 1:9 - 4:1)* | | **Metronidazole - Ibuprofen** (1:1 ideal and complete reaction) | |
| | | | **Triclosan** - **Ibuprofen** (1:1 complete reaction with liquid portions measurable across entire composition range) | |
| **Local anaesthetics** *possessing amide groups* | **Lidocaine - Capric acid** (1:4 - 3:2) | **Lidocaine** - **1-Decanol** (=< 1:2) | **Lidocaine** - **Ibuprofen** (3:7-7:3) | |
| | **Lidocaine - Hexanoic acid** (1:6 - 4:1) | **Lidocaine** - **1-dodecanol** (1:1) | | |
| | | | **Lidocaine** - **Ketoprofen** (1:1) | |
| | **Lidocaine - Lauric acid** (1:1) | **Lidocaine** - **1-tetradecanol** (1:1) | | |
| | **Lidocaine - Adipic acid** (1:5 - 3:1) | **Lidocaine** - **3-Methyl-3-Pentanol** (1:1) **(3MetPen)** | | |
| | **Lidocaine - Sebacic acid** (2:3 - 3:1) | | | |
| | **Lidocaine - Myristic acid** (1:1) | | | |
| | **Lidocaine** - **Dodecanedioic acid** (1:1 - 3:1) | | | |
| | **Lidocaine - Tetradecanedioic acid** (1:1) | | | |

| | | | | |
|---|---|---|---|---|
| ***Note the compositions are written as molar ratios in this table.** | | | | |

### Experimental Results

1. Drug-incorporated/eluting surgical sutures, manufacturable using an end-to-end continuous reactive extrusion technology, comprising therapeutic deep eutectic systems (THEDES) for improved surgical site wound management.
2. The THEDES embedded within the matrices of the developed suture threads might be composed of:
   a) H-bonded (charge-assisted) molecular complexes between an antibiotic (i.e. metronidazole) and a fatty acid (i.e. maleic acid) at molar ratios that promote complete reaction of all molecules (case-variant dependent upon the number of active functional groups, equimolar for metronidazole and maleic acid);
   b) H-bonded (charge-assisted) molecular complexes between a non-antibiotic antimicrobial (NAAM, i.e. triclosan) and a carboxylic acid non-steroidal anti-inflammatory drug (NSAID, i.e. ibuprofen) at molar ratios that promote complete reaction of all molecules (case-variant dependent upon the number of active functional groups, equimolar for triclosan and ibuprofen).
3. The THEDESs embedded within the matrices of the developed suture threads showing significantly improved antibacterial efficacies against commonly seen bacterial species (both gram-positive and gram-negative), compared to each individual parent compound.
4. The extruded suture threads embedded with THEDESs were within a size range of approx. 200 - 249 microns, equivalent to that of a USP #3-0 suture.
5. The suture threads embedded with THEDESs were capable of accommodating significantly increased active compound contents (extrudable up to 50 wt%), when compared with drug-eluting sutures either from the literature or available commercially (typical drug loading not more than 20 wt%).
6. The suture threads embedded with THEDESs showing significantly enhanced antimicrobial efficacies (great areas measurable from zone of inhibition studies), when compared with suture threads loaded with each respective parent compound at the same drug loading.
7. The suture threads embedded with THEDESs showing significantly less negative impact on mechanical characteristics (tensile failure force), when compared with suture threads loaded with each respective parent compound at the same drug loading.
8. The suture threads embedded with THEDESs showing significantly increased rate and extent of drug release, when compared with suture threads loaded with each respective parent compound at the same drug loading.
9. The suture threads embedded with THEDESs showing controlled drug release profile with rapid onset of release followed by continuous replenishment throughout the intended duration of action (7-14 days variant dependent upon targeted site of application through adjustment of the biodegradation profile).

### Technical Details

In this work, a first-line choice antibiotic agent (Metronidazole) in both pre-surgical prophylaxis and post-surgery wound management was employed in combination with a short-chain fatty acid (SCFA) with reported antimicrobial activities. The formation of an equimolar THEDES between metronidazole (MET) and maleic acid (MA) has been confirmed successful during reactive extrusion processing in the presence of a polymeric carrier polycaprolactone. ATR-FTIR was carried out on the extruded sutures to establish if formation of the THEDES complex was successful within the extrudate suture threads and if any interactions were occurring between the THEDES parent compounds and PCL, interfering with formation of the THEDES.

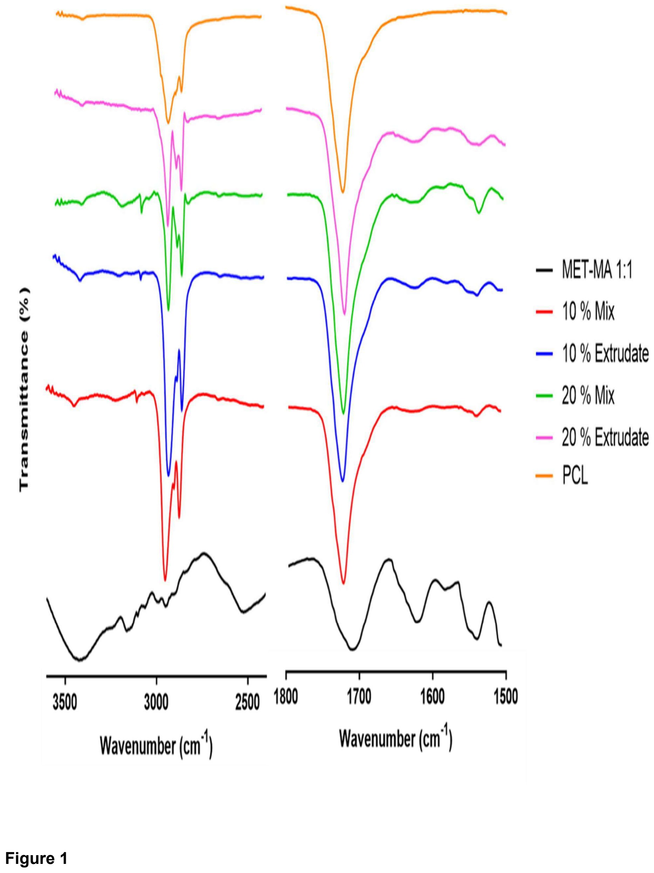

As shown in Figure 1, characteristic peaks of the pure equimolar MET-MA THEDES present at 3427, 1712 and 1580 cm⁻¹, respectively. The band at 3427 cm⁻¹ in the neat THEDES, which was attributed to free MET O-H, presents as a small band in the extrudates at 3446 cm⁻¹. The MA C=O shifting to 1712 cm⁻¹ is masked by the very strong C=O band from PCL, but the 1580 cm⁻¹ band indicative of the ionised COO⁻ stretch can be observed in the MET-MA-PCL extrudates as a small band at 1577 cm⁻¹.

These results confirmed that THEDES formation was indeed successful in PCL, with non-detectable interfering interactions between the parent components, the THEDES and the carrier.

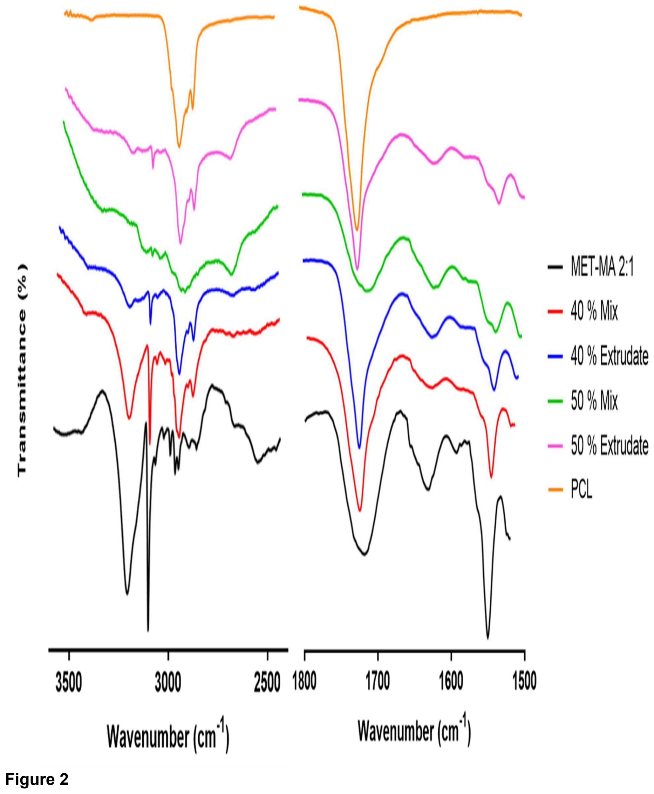

Similar results were observed in the extruded PCL matrix comprising a MET-MA 2:1 mixture, with peaks representing the THEDES and excess MET both being observed (Figure 2).

Characteristic bands of the THEDES were again observed at wavenumbers of 3444 cm⁻¹ and 1581 cm⁻¹, with the peak at 1712 cm⁻¹ again being masked by the PCL C=O band. Bands characteristic of pure MET were also observed, such as the intra-molecularly H-bonded O-H stretch at 3210 cm⁻¹, the aromatic C-H stretch at 3100 cm⁻¹, and the N-O asymmetric stretches at 1534 cm⁻¹ and 1485 cm⁻¹.

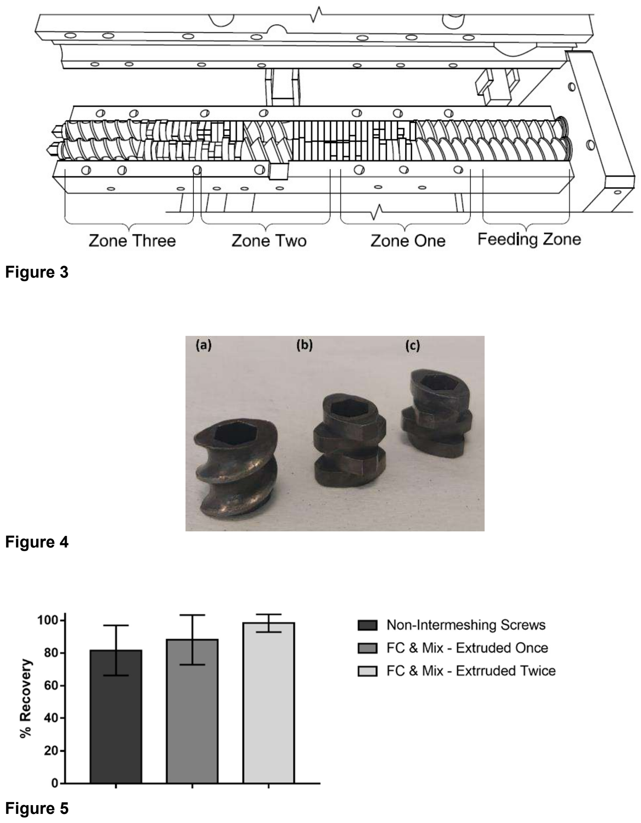

Reactive extrusion was performed using a Rondol Microlab (L/D 20:1) intermeshing twin-screw co-rotating compounder (Figure 3) equipped with a range of screw elements (Figure 4) for sufficient conveying/kneading/discharging.

Formulations were found to exhibit improved content uniformity with narrower deviation (Figure 5) when processed twice for increased residence time within the extrusion barrel.

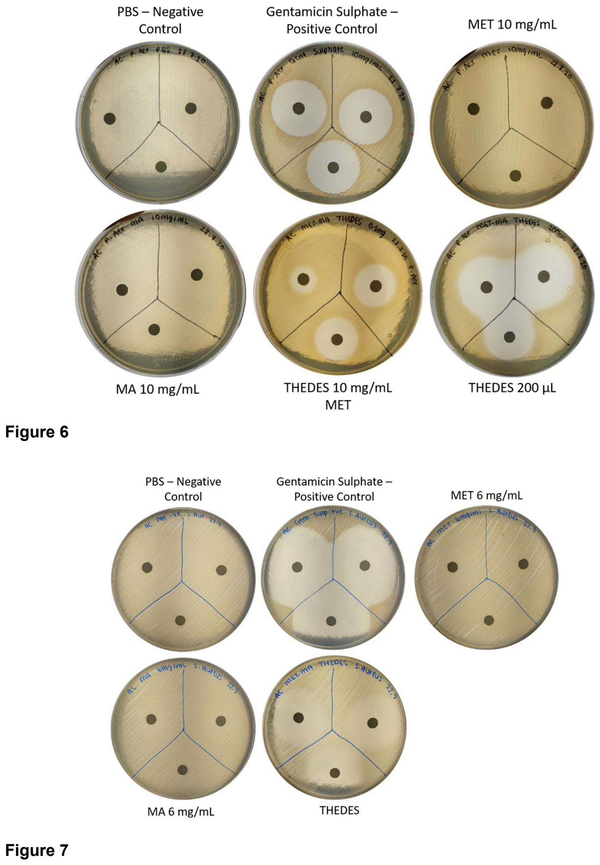

Disc diffusion inhibition zone assay has found that the equimolar metronidazole-maleic acid THEDES showed a superior efficacy against *Pseudomonas aeruginosa,* one of the most common pathogens found on surgical wound sites, with areas of the inhibition zones comparable to that generated by the gentamicin sulphate positive control (Figure 6). Both parent agents, on the other hand, showed identical negative outcomes as the blank PBS negative control.

To achieve a more comprehensive understanding of the THEDES's antimicrobial efficacy, a wide variety of additional bacterial strains were canvased in later study; namely, *Escherichia coli, Enterococcus faecalis, Klebsiella pneumoniae, Staphylococcus epidermidis* and *Staphylococcus aureus,* chosen due to their prevalence in wound infections. Particular attention was paid to the antimicrobial activity of the THEDES with *P. aeruginosa,* as patients with SSIs containing *P*. *aeruginosa* commonly undergo extended durations of antibiotic treatment, with worse outcomes observed for patients overall. Furthermore, *K. pneumoniae* is one of the most frequently identified bacterium presents in SSIs following appendectomy procedures. A study on SSIs following colorectal surgery detected a variety of organisms present in this work, including S. *aureus,* E. *faecalis, E. coli,* and *K. pneumoniae.*

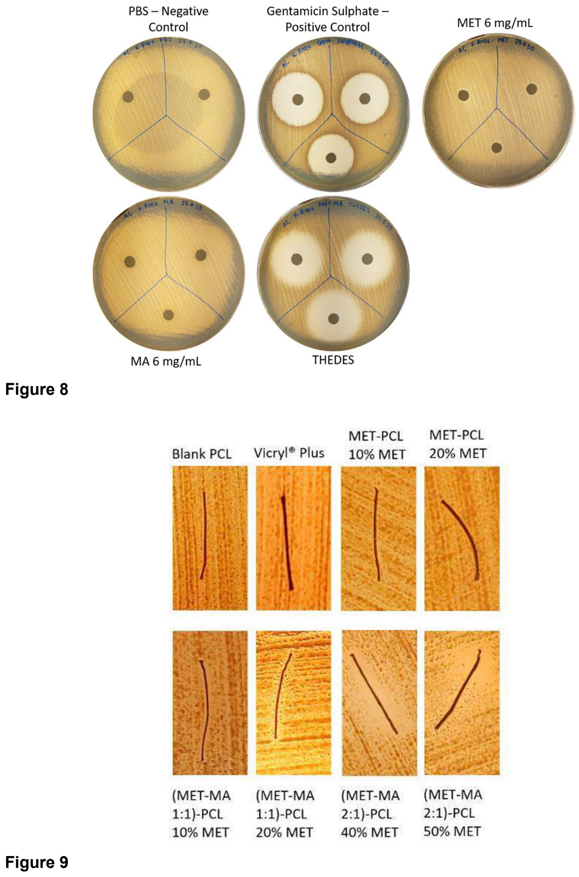

The disc diffusion assays were carried out against *Staphylococcus aureus* (Figure 7, Gram-positive, frequently found in the upper respiratory tract and on the skin) and *Klebsiella pneumoniae* (Figure 8, Gram-negative, facultative anaerobic, typically residing in human intestines and faeces). In general, the calculated inhibition zones of gentamicin sulphate (10 mg/mL) and the neat THEDES were very similar, confirming the antimicrobial activity of the THEDES against both gram-positive and gram-negative bacteria (Table 3). Such results are extremely significant as metronidazole is highly active against gram-negative bacteria, whilst fatty acids and their derivatives, on the other hand, are known to work against gram-positive bacteria. The combination of both, in the form of a THEDES, demonstrated a dual synergistic antimicrobial activity against both gram-negative and gram-positive species, broadening its potential applicability.

**Table 3 Zone of inhibition (ZOI) sizes for P. Aueruginosa, S. Aureus and K. Pneumoniae after being challenged with gentamicin sulphate (10 mg/mL) and the neat MET-MA THEDES. Results are shown as the average (n = 9) ± S.D.**

| | Gentamicin Sulphate ZOI Diameter (mm) | MET-MA THEDES ZOI Diameter (mm) |
|---|---|---|
| *P. Aeruginosa* | 26.04 ± 1.75 | 20.84 ± 4.11 |
| *S*. *Aureus* | 46.09 ± 1.69 | 24.03 ± 1.17 |
| *K. Pneumoniae* | 24.13 ± 1.09 | 26.99 ± 1.54 |

The antimicrobial activity of the THEDES was also assessed by determining the minimum inhibitory concentration (MIC) and the minimum bactericidal concentration (MBC) of MET, MA and the equimolar THEDES, respectively. Concentrations of 6 mg/mL MET, MA and MET-MA 1:1 were used to determine the MIC and MBC values against gram-positive bacteria E. *faecalis, S. epidermidis* and *S*. *aureus,* and gram-negative bacteria *P. aeruginosa, E. coli* and *K. pneumoniae;* results are shown in Table 4. In all cases, the positive control exhibited microbial growth and the negative control did not.

**Table 4 MIC and MBC results against a variety of bacterial strains, for MET, MA and the MET-MA THEDES.**

| Bacterial species | | MET (µg/mL) | MA (µg/mL) | MET-MA (µg/mL) |
|---|---|---|---|---|
| E. faecalis ATCC 29212 | MIC | > 6000 | 6000 | 6000 |
| | MBC | > 6000 | > 6000 | > 6000 |
| S. epidermidis ATCC 35984 | MIC | 3000 | 6000 | 1500 |
| | MBC | 3000 | > 6000 | 3000 |
| S. aureus ATCC 29213 | MIC | 6000 | 6000 | 6000 |
| | MBC | > 6000 | > 6000 | 6000 |
| S. aureus NCTC 10788 | MIC | 6000 | 6000 | 6000 |
| | MBC | > 6000 | > 6000 | 6000 |
| S. aureus NCTC 12493 (MRSA) | MIC | > 6000 | 6000 | 6000 |
| | MBC | > 6000 | > 6000 | 6000 |
| P. aeruginosa NCTC 10783 | MIC | > 6000 | 6000 | 6000 |
| | MBC | > 6000 | > 6000 | 6000 |
| P. aeruginosa ATCC 27853 | MIC | > 6000 | 6000 | 6000 |
| | MBC | > 6000 | > 6000 | > 6000 |
| E. coli NCTC 8196 | MIC | > 6000 | 6000 | 6000 |
| | MBC | > 6000 | > 6000 | 6000 |
| K. pneumoniae ATCC 700603 | MIC | 6000 | > 6000 | 3000 |
| | MBC | 6000 | > 6000 | 3000 |

A major point to note, is that for the THEDES, the MIC and MBC value obtained either matched the lowest value from either parent component or was indeed lower than each. This signified the retention of the antimicrobial activity of MET when provided as a THEDES, and also the enhanced activity as a result of this complexation with MA. This occurred in several bacterial strains, such as *E. faecalis,* several *S. aureus* strains, both *P. aeruginosa* strains and *E. coli.* The methicillin-resistant strain of *S. aureus* (MRSA) was shown to show a greater resilience to the antimicrobial activity of pure MET, with an MIC of 6 mg/mL for both non-methicillin-resistant strains and > 6 mg/mL for the methicillin-resistant strain.

Similar to the disc diffusion assays described above, zone of inhibition (ZOI) studies were carried out using suture lengths to further evaluate if the THEDES-loaded suture possessed an enhanced antimicrobial activity when compared to sutures containing pure MET. Figure 9 shows the ZOls formed against *P. aeruginosa* by MET, the MET-MA THEDES and MET-MA in a 2:1 ratio, which included the THEDES with excess MET.

*P. aeruginosa,* a gram-negative bacterium, was chosen to be investigated in this study, as it is the fourth leading cause of healthcare-associated infections worldwide. As this bacterium is frequently found in patients with underlying conditions, it is regularly associated with a high rate of mortality due to late prognosis. No zone was observed for the control, which was expected due to the lack of antimicrobial agent. No zone was also observed for the marketed suture, Vicryl^{®} Plus, which contains triclosan (TRC). The size of the zones was observed to increase with increasing concentrations of MET, indicating a concentration-dependent effect. This also demonstrated that MET had the ability to diffuse out of the suture in order to kill microbial cells. Again, the THEDES demonstrated an enhanced antimicrobial activity against *P. aeruginosa* than MET, demonstrated by the larger ZOls obtained (Table 5).

**Table 5 Average ZOI height (mm), width (mm) and area (mm²) for (MET-MA 1:1)-PCL 10 % and 20 % w/w MET and (MET-MA 2:1)-PCL 40 % and 50 % w/w MET, against P. aeruginosa. Numbers are shown as the average ± S.D., where n = 9.**

| | (MET-MA 1:1)-PCL 10% MET | (MET-MA 1:1)-PCL 20% MET | (MET-MA 2:1)-PCL 40% MET | (MET-MA 2:1)-PCL 50% MET |
|---|---|---|---|---|
| Zone height (mm) | 10.07 ± 0.06 | 10.26 ± 0.14 | 10.36 ± 0.22 | 10.73 ± 0.42 |
| Zone width (mm) | 0.96 ± 0.14 | 1.11 ± 0.14 | 1.92 ± 0.51 | 3.15 ± 0.62 |
| Zone area (mm²) | 9.66 ± 1.43 | 11.36 ± 1.38 | 19.89 ± 5.30 | 33.69 ± 5.78 |

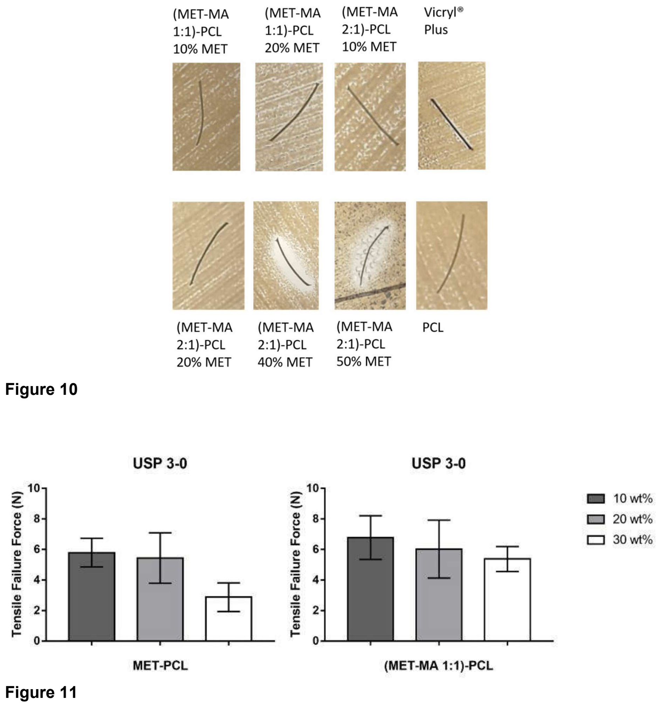

Similar results were also observed for *K. pneumoniae.* After 24 h incubation, ZOls were observed for the two sutures which contained THEDES and excess MET; namely, the (MET-MA 2:1)-PCL matrices containing 40 % and 50 % w/w MET (Figure 10 and Table 6). Higher concentrations of MET were required to elicit a bactericidal effect against this bacterium from the suture when compared with zones obtained for P. aeruginosa.

**Table 6 Average ZOI height (mm), width (mm) and area (mm2) for (MET-MA 2:1)-PCL 40 % and 50 % w/w MET, against K. pneumoniae. Numbers are shown as the average ± S.D., where n = 9.**

| | (MET-MA 2:1)-PCL 40 % | (MET-MA 2:1)-PCL 50 % |
|---|---|---|
| Zone height (mm) | 10.50 ± 0.12 | 11.21 ± 0.57 |
| Zone width (mm) | 2.20 ± 0.73 | 2.86 ± 0.74 |
| Zone area (mm²) | 23.11 ± 7.96 | 32.37 ± 9.57 |

The use of a suture is strongly dependent on the mechanical strength of the material, and as such is one of the most widely reported properties of sutures, with it being essential that there is a proper correlation between the strength of the suture and the strength of the tissue into which it will be implanted. Tensile strength analysis was carried out here in order to compare the force at break of sutures which were MET-loaded, THEDES-loaded and a mixture of MET- and THEDES-loaded. Traditionally, the maximum amount of solid API able to be loaded into a suture using HME and electrospinning is 20 % w/w API, due to the loss of mechanical properties with increasing solid content throughout the polymer matrix. As a result, the effect of tensile strength through incorporation of a liquid THEDES and mixture of THEDES and solid MET into a suture was investigated here.

The incorporation of the THEDES did not significantly affect the strength of the suture at drug loadings of 10 % and 20 % w/w MET, but was successful in significantly increasing the mechanical strength at a higher loading of 30 % w/w MET, seen through the greater tensile failure force (N) (Figure 11). The highest average tensile failure force was for the (MET-MA 1:1)-PCL 10 % w/w MET suture, at 6.8 ± 1.4 N.

In order to determine the release profile of MET when embedded in PCL as a suture material, PBS (pH 7.4) was used as the dissolution medium at 37 ± 0.5 °C, simulating physiological conditions.

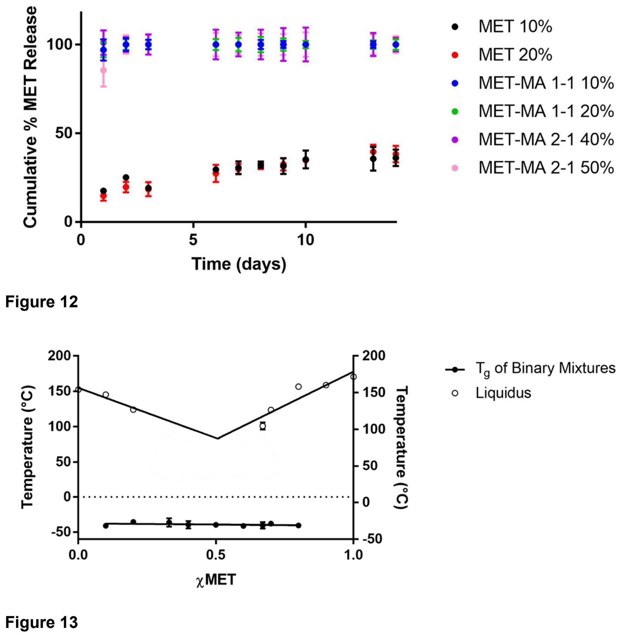

The drug release profile (Figure 12) compared the release behaviour of extruded suture threads containing MET, THEDES and a MET-THEDES mixture, respectively. It can be seen that, when present in the liquid state (as THEDES or a mixture of MET-THEDES), a rapid onset and completion of MET release was shown. A much slower release of MET was observed from extruded matrices containing solid MET, where after 14 days, only 35.6 ± 6.7 % and 38.3 ± 4.6 % of MET were released from the 10 % and 20 % w/w MET matrices, respectively (Table 7).

**Table 7 Drug percentage (DP %) at particular timepoints for the data presented in Figure 12.**

| | DP (%) | | |
|---|---|---|---|
| | Day 1 | Day 7 | Day 14 |
| MET 10 % | 17.5 ± 1.3 | 30.6 ± 3.6 | 35.6 ± 6.7 |
| MET 20 % | 14.8 ± 2.8 | 29.8 ± 3.0 | 38.3 ± 4.6 |
| MET-MA 10 % | 97.0 ± 6.0 | 100.1 ± 0.9 | 100.0 ± 1.2 |
| MET-MA 20 % | 97.0 ± 4.1 | 100.1 ± 3.7 | 100.1 ± 3.0 |
| MET-MA 40 % | 101.1 ± 6.9 | 100.1 ± 6.7 | 100.6 ± 3.8 |
| MET-MA 50 % | 85.5 ± 9.1 | 101.1 ± 5.1 | 100.1 ± 4.7 |

Further information is now provided on various eutectic forming binary components, with reference to Table 2 above.

Figure 13 is a temperature-molar ratio phase diagram of Metronidazole-Maleic acid binary system, with liquidus shown as open circles and Tg of the binary mixtures shown as filled circles; each plot is the average of three replicates (mean ± S.D.). The solid line indicates linear extrapolation used to identify the theoretical eutectic composition. At compositions where melting event is absent, the formed is a room temperature liquid eutectic system.

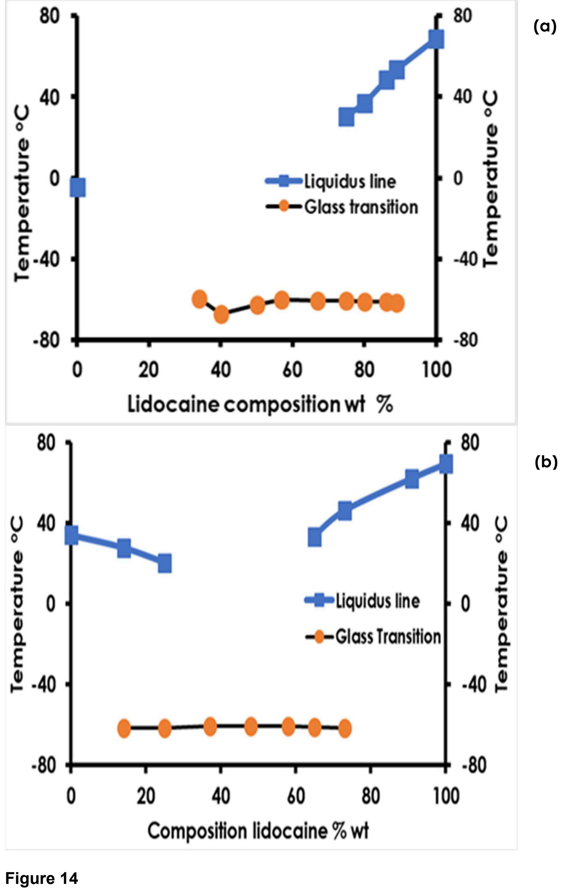

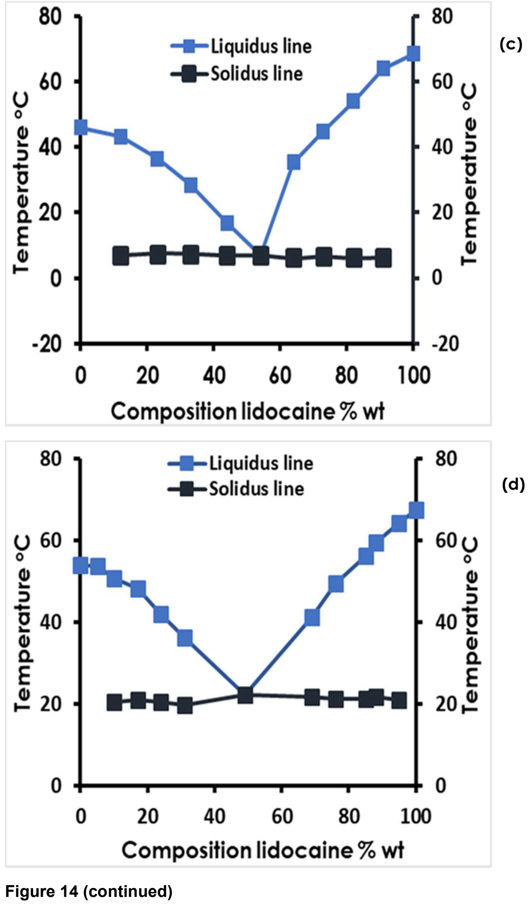

Figure 14 shows DSC phase diagrams for (a) Lidocaine - Hexanoic acid, (b) Lidocaine - Capric acid, (c) Lidocaine - Lauric acid and (d) Lidocaine - Myristic acid binary mixtures at varying compositions, showing formation of room temperature liquid (deep) eutectic systems at compositions where either liquidus line is missing, or the intersected eutectic composition indicates a eutectic melting point that is below room temperature.

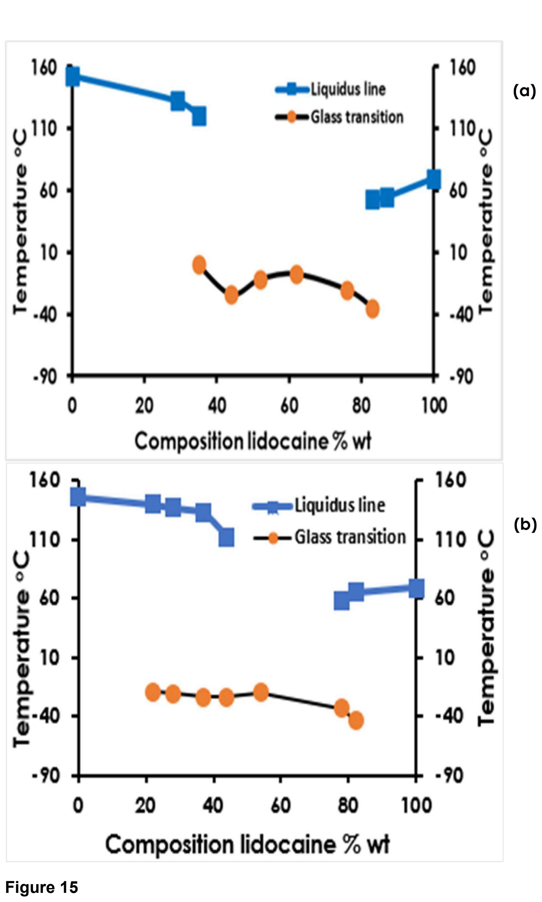

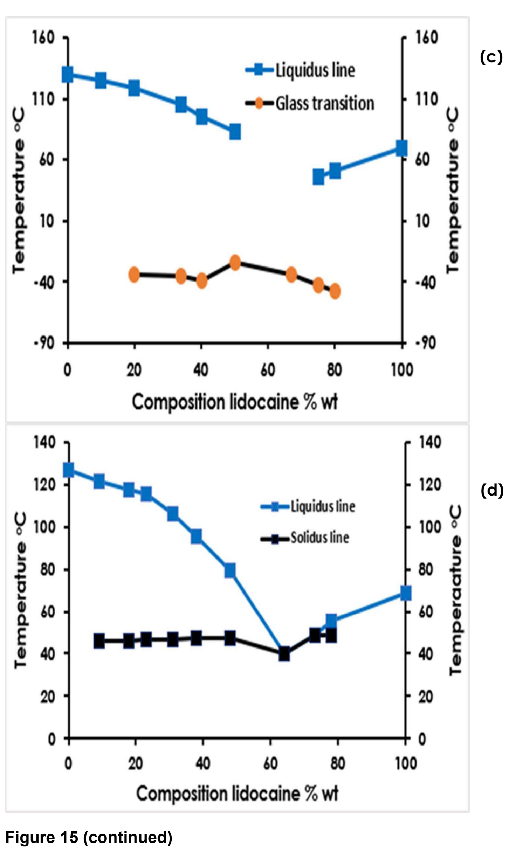

Figure 15 shows DSC phase diagrams for (a) Lidocaine - Adipic acid, (b) Lidocaine - Sebacic acid, (c) Lidocaine - Dodecanedioic acid and (d) Lidocaine - Tetradecanedioic acid binary mixtures at varying compositions, showing formation of room temperature liquid (deep) eutectic systems at compositions where either liquidus line is missing, or the intersected eutectic composition indicates a eutectic melting point that is below room temperature.

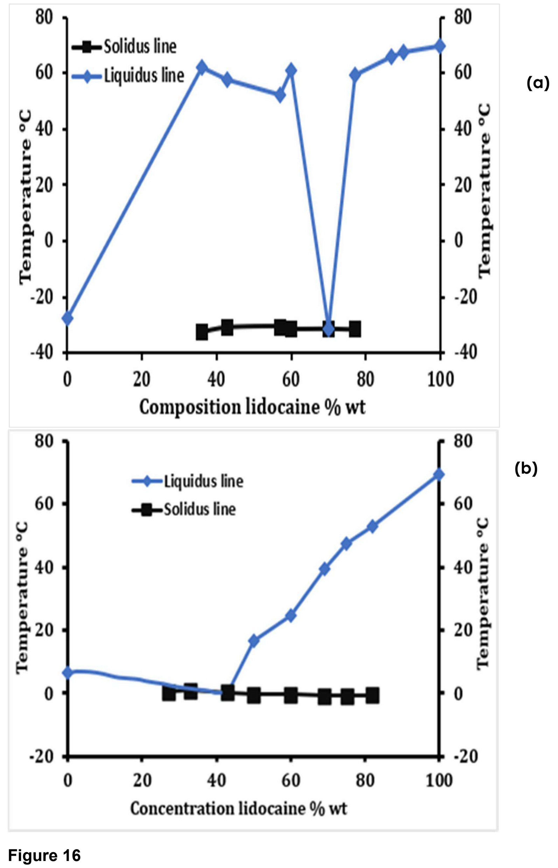

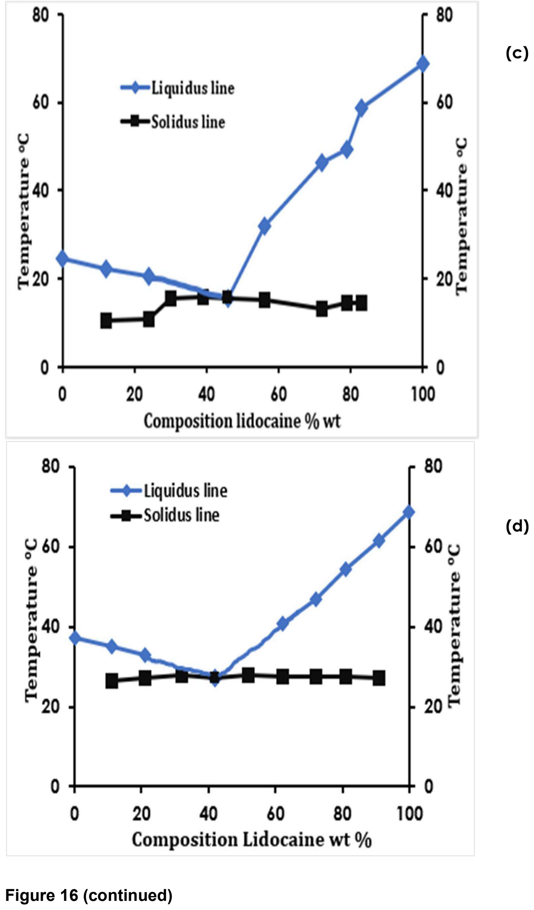

Figure 16 shows DSC phase diagrams for (a) Lidocaine - 3MetPen, (b) Lidocaine - 1-decanol, (c) Lidocaine - 1-dodecanol and (d) Lidocaine - 1-tetradecanol binary mixtures at varying compositions, showing formation of eutectic systems with eutectic melting points below room temperature.

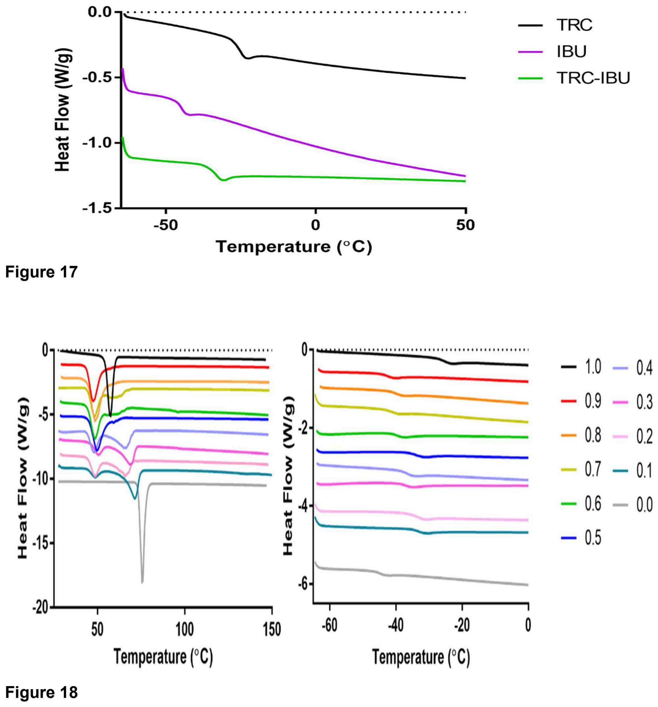

Figure 17 shows DSC thermogram of triclosan (TRC), ibuprofen (IBU) and an equimolar (1:1) physical mixture of TRC-IBU in the region of -65-50 °C on the second heat curve of a heat-cool-heat cycle. The equimolar binary mixture (the lowest trace on Figure 17) formed a miscible system characterised by a single glass transition (Tg) on the second heat curve between the individual glass transition events of the two parent components. This formed equimolar mixture was observed to be a clear RT liquid.

Figure 18 shows DSC thermograms of (from top to bottom) triclosan and ibuprofen, TRC-IBU _{X}TRC = 0.9, 0.8, 0.7, 0.6, 0.5, 0.4, 0.3, 0.2 and 0.1, physical mixtures (right hand drawing) and after heating at 10 °C/min to 200 °C (left hand drawing). The Tg of each TRC-IBU mix is also seen on the second heat. It can be seen that, upon heating physical mixtures of TRC and IBU, a liquid portion can be detected in the mixture across the entire composition range.

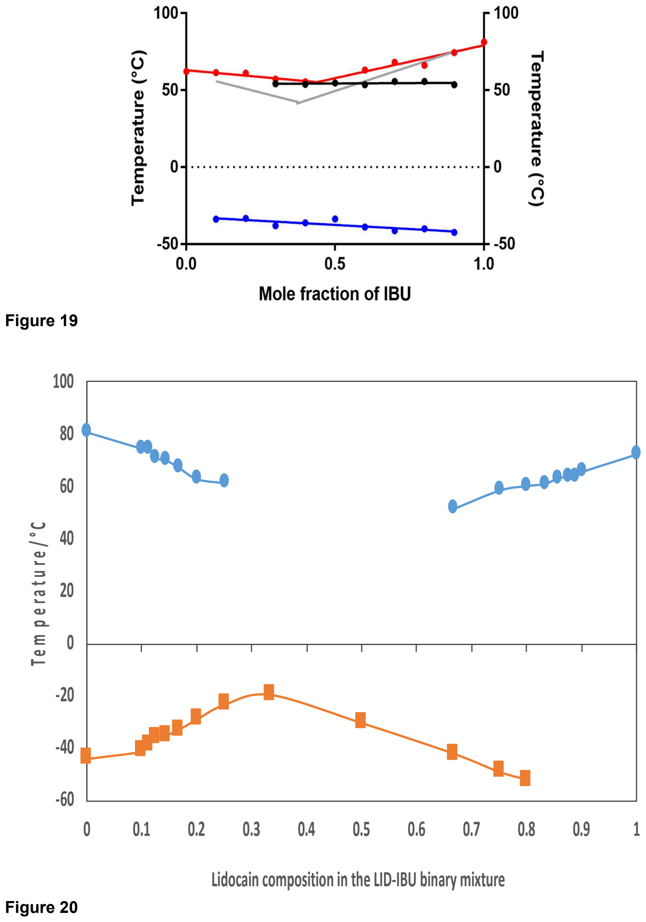

Figure 19 shows phase diagram of the TRC-IBU system, where the red dots represent the liquidus, the black dots represent the solidus and the blue dots show the glass transition of the coamorphous system. The grey lines represent the theoretical liquidus lines, calculated using the Schröder-Van Laar equation. Although showing a eutectic temperature of 47.75 ± 2.13 °C (peak of the melting events), these eutectic systems were observed to be clear liquids after cooled down to room temperature from a thermal preparation.

Figure 20 shows temperature-molar ratio phase diagram of lidocaine-ibuprofen (LID-IBU) binary system, each plot is the average of three replicates (mean ± S.D.). At compositions where melting event is absent (0.3-0.7), the formed is a room temperature liquid eutectic system.

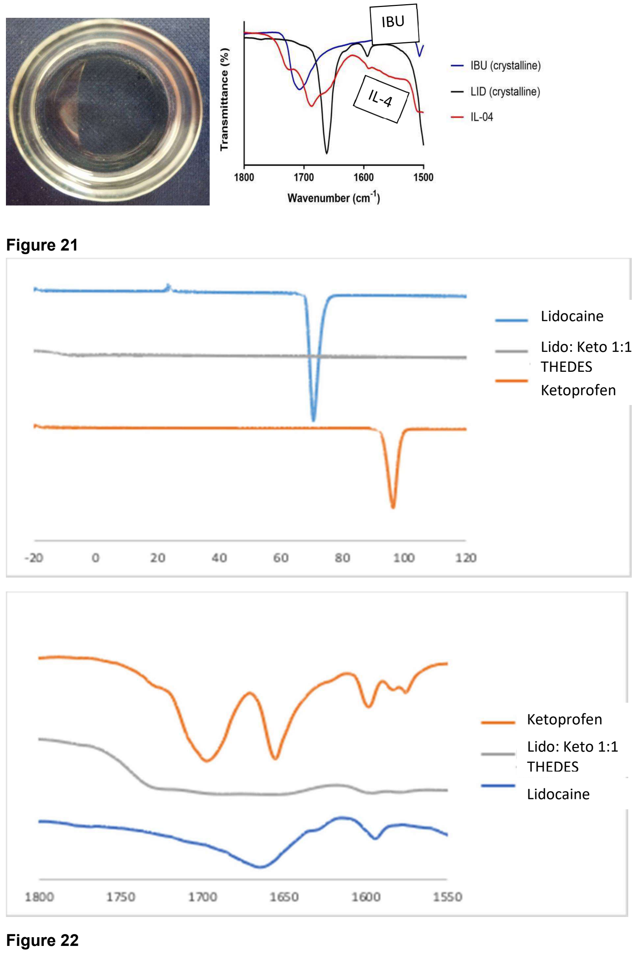

*Figure 21* *shows (left)* LID-IBU equimolar mixture extruded, without polymer, at 60°C showing clear RT liquid (deep) eutectic formation. *(right)* extruded LID-IBU equimolar THEDES exhibiting characteristic charge-assisted H-bonding between the carboxylic acid group from IBU and the amide group from LID.

Figure 22 shows equimolar lidocaine and ketoprofen. THEDES formation is indicated by DSC (complete loss of melting events) and FTIR (characteristic charge-assisted H-bonding pattern between LID amide and ketoprofen carboxylic acid).

### Embodiments

1. A suture thread comprising
   two or more eutectic forming components selected from an acid, a fatty acid, a fatty amine, a fatty alcohol, or any combination of two or more thereof that are capable of forming, *in situ,* at least one eutectic mixture in or on at least one polymer.
2. A suture thread as described in embodiment 1 wherein the one or more of the eutectic forming components comprises one or more active agents.
3. A suture thread as described in embodiment 1 or 2 wherein the one or more of the eutectic forming components comprises a Lewis or Brønsted-Lowry acid and base.
4. A suture thread as described in embodiment 1 or 2 comprising:
   wherein the at least one polymer is at least one biocompatible polymer;
   wherein, optionally, the at least one biocompatible polymer is at least one hot melt extrudable biocompatible polymer.
5. A suture thread as described in any of the preceding embodiments wherein the two or more eutectic forming components comprises a first active agent and one or more components from the second group that are capable of forming a eutectic mixture with the first active agent, the second group comprising
   i) one or more second active agents; or
   ii) one or more acids; or
   iii) one or more fatty acids; or
   iv) one or more fatty alcohols; or
   v) one or more fatty amines; or
   any combination of i) to iv) above, including any combination of two or more of i) to v).
6. A suture thread as described in embodiment 5, wherein the first active agent is a Lewis or Brønsted-Lowry acid or base; and the component from the second group is the other of the Lewis or Brønsted-Lowry acid and base pair.
7. A suture thread as described in embodiment 5 or 6 wherein the first and / or second active agent is at least one of an antibacterial, an antifungal, an antiviral, an anti-inflammatory or a local anesthetic .
8. A suture thread as described in embodiment 7, wherein the local anesthetic comprises functional groups with strong group electronegativity that is an amide selected from lidocaine, prilocaine, and bupivacaine or an ester selected from procaine, benzocaine and tetracaine.
9. A suture thread as described in embodiment 7, wherein the antibacterial, an antifungal, an antiviral, or an anti-inflammatory is selected from antibiotics, non-antibiotic antimicrobials, antivirals, antifungals and antiparasitics comprising an imidazole or nitroimidazole.
10. A suture thread as described in any of the preceding embodiments wherein the polymer is selected from the group comprising polycaprolactone , polyglycolic acid (PGA), polylactic co-glycolic acid (PLGA), polylactic acid (PLA), polydioxanone, poliglecaprone (copolymer of glycolide and epsilon-caprolactone), poly-(trimethylene carbonate)-based polymers, polypropylene, polyester , and nylon.
11. A suture thread as described in any of the preceding embodiments wherein the polymer is biodegradeable.
12. A suture thread as described in any of the preceding embodiments wherein the polymer is polycaprolactone.
13. A suture thread as described in any of embodiments 1 to 10 wherein the polymer is substantially non-biodegradeable.
14. A suture thread as described in any of embodiments 1 to 10 or 13 wherein the polymer is PLGA.
15. A suture thread as described in any of the preceding embodiments wherein the eutectic mixture comprises maleic acid and an antibiotic.
16. A suture thread as described in any of embodiments 1 to 14 wherein the eutectic mixture comprises maleic acid and a non-antibiotic antimicrobial (NAAM).
17. A suture thread as described in any of embodiments 1 to 14 wherein the eutectic mixture comprises a non-antibiotic antimicrobial (NAAM) and a non-steroidal anti-inflammatory (NSAIDs).
18. A suture thread as described in any of the preceding embodiments wherein the two or more eutectic forming components generate eutectic oils in situ within a polymer device.
19. A suture thread as described in any of embodiments 1 to 16 wherein the eutectic mixture comprises at least one fatty acid.
20. A suture thread as described in any of embodiments 1 to 16 and 19 wherein the eutectic mixture comprises at least one fatty acid that is a mono or dicarboxylic, saturated or unsaturated, fatty acid comprising 4 to 20 carbon atoms.
21. A suture thread as described in any of embodiments 1 to 16, 19 and 20 wherein the fatty acid is selected from the group consisting of maleic acid, hexanoic acid, hexanedioic acid, capric acid, decanedioic acid, lauric acid (C12), dodecanedioic acid, tetradecanedioic acid, myristic acid, and oleic acid.
22. A suture thread as described in any of embodiments 1 to 14 wherein the eutectic mixture comprises at least one NSAIDs comprising a carboxylic acid functionality.
23. The suture thread of embodiment 22 wherein the at least one NSAIDs comprising a carboxylic acid functionality is selected from derivatives of propionic acid, derivatives of acetic acid and derivatives of anthranilic acid.
24. The suture thread of embodiment 22 or 23 wherein the at least one NSAIDs comprising a carboxylic acid functionality is selected from the group consisting of ibuprofen, naproxen, indomethacin, diclofenac, mefenamic acid, meclofenamic acid.
25. A suture thread as described in any of the preceding embodiments where the eutectic mixture comprises a fatty acid and a second fatty acid, a fatty alcohol, a fatty amine or an active agent or other agent.
26. A suture thread as described in any of embodiments 1 to 14 where the eutectic mixture comprises an antibiotic.
27. A suture thread las described in any of embodiments 1 to 14 wherein the eutectic mixture comprises metronidazole.
28. A suture thread as described in any of embodiments 1 to 14 wherein the eutectic mixture comprises a non-antibiotic antimicrobial.
29. A suture thread as described in any of embodiments 1 to 14 wherein the eutectic mixture comprises triclosan.
30. A suture thread as described in any of embodiments 1 to 14 wherein the eutectic mixture comprises metronidazole and ibuprofen.
31. A suture thread as described in any of embodiments 1 to 14 wherein the eutectic mixture comprises triclosan and ibuprofen or ketoprofen.
32. A suture thread as described in any of embodiments 1 to 14 wherein the polymer is polycaprolactone and the eutectic mixture comprises maleic acid and metronidazole.
33. A suture thread as described in any of embodiments 1 to 14 wherein the polymer is PLGA and the eutectic mixture comprises maleic acid and metronidazole.
34. A suture thread as described in any of embodiments 1 to 14 wherein the polymer is polycaprolactone and the eutectic mixture comprises metronidazole and ibuprofen.
35. A suture thread as described in any of embodiments 1 to 14 wherein the polymer is polycaprolactone and the eutectic mixture comprises triclosan and ibuprofen or ketoprofen.
36. A suture thread as described in any of embodiments 1 to 14 wherein the polymer is PLGA and the eutectic mixture comprises metronidazole and ibuprofen.
37. A suture thread as described in any of embodiments 1 to 14 wherein the polymer is PLGA and the eutectic mixture comprises triclosan and ibuprofen or ketoprofen.
38. A suture thread as described in any of the preceding embodiments wherein the thread size (diameter) ranges from # 4-0 to # 4 according to the USP standard.
39. A suture thread as described in any of the preceding embodiments wherein the biodegradation profile ranges between 7-30 days.
40. A suture thread as described in any of embodiments 1 to 16 wherein the eutectic mixture comprises a fatty alcohol.
41. A suture thread as described in embodiment 40 wherein the fatty alcohol is a straight-chain or branched-chain, saturated or unsaturated primary alcohol, ranging from 4-6 carbons to as 26-30 carbons.
42. A suture thread as described in embodiment 40 or 41 wherein the fatty alcohol is selected from 3-methyl-3-pentanol ; 1-Dodecanol, Heneicosanol, Elaidyl alcohol, Petroselinyl alcohol, 1-tetradecanol, 1-Docosanol, 1-Tridecanol, 1-Nonadecanol, 1-Triacontanol, 1-Pentadecanol, 1-Nonanol, 1-Tricosanol, Cetyl alcohol, Stearyl alcohol, 1-Decanol, or Isooctyl alcohol.
43. A suture thread as described in any of embodiments 1 to 16 wherein the eutectic mixture comprises a fatty amine.
44. A suture thread as described in embodiment 43 wherein the fatty amine is a straight-chain or branched-chain, saturated or unsaturated amine.
45. A suture thread as described in embodiment 43 or 43 wherein the fatty amine is selected from a mono- or di-substituted amine that is optionally substituted with a short chain alkyl at the amino group.
46. A suture thread as described in any of embodiments 43 to 45 wherein the fatty amine comprises from 4-6 carbons to 22-26 carbons.
47. A suture thread as described in any of embodiments 43 to 46 wherein the fatty amine is selected from Pentadecylamine, Hexadecylamine, Docecylamine, Decylamine, Tridecylamine, Octadecylamine, Undecylamine, N, N-dimethyltetradecylamine, Dodecylamine, or Octadecylamine.
48. Eutectic mixtures for use in suture threads to reduce surgical site infections (SSis) and/or to decrease pain and/or to increase lubricity of sutures.
49. Eutectic mixtures for use of embodiment 48 wherein two or more eutectic forming components selected from an acid, a fatty acid, a fatty amine, a fatty alcohol, or any combination of two or more thereof form the eutectic mixture, *in situ.*
50. Eutectic mixtures for use of embodiment 49 wherein the two or more eutectic forming components form, *in situ,* the at least one eutectic mixture in or on at least one polymer.
51. A method for the production of sutures of any of embodiments 1 to 50 with drug loadings.
52. A method for the production of sutures with drug loadings, the method comprising the use of therapeutic deep eutectic solvent (THEDES) technology.
53. The method of embodiment 51 or 52, wherein two or more eutectic forming components selected from an acid, a fatty acid, a fatty amine, a fatty alcohol, or any combination of two or more thereof that are capable of forming, *in situ,* at least one eutectic mixture are co-extruded with at least one polymer at a temperature above the melting point of the polymer.
54. A suture thread as described in any of embodiments 1 to 14 wherein the eutectic mixture comprises triclosan and lidocaine.

## Claims

1. A suture thread comprising
two or more eutectic forming components selected from an acid, a fatty acid, a fatty amine, a fatty alcohol, or any combination of two or more thereof that form, *in situ,* at least one eutectic mixture in or on at least one polymer;
wherein the two or more eutectic forming components comprise one or more active agents;
wherein the two or more eutectic forming components comprise a first active agent and one or more components from a second group that are capable of forming a eutectic mixture with the first active agent, the second group comprising
i) one or more second active agents; or
ii) one or more acids; or
iii) one or more fatty acids; or
iv) one or more fatty alcohols; or
v) one or more fatty amines; or
any combination of i) to iv) above, including any combination of two or more of i) to v);
wherein the first and/or second active agent is at least one of an antibacterial, an antifungal, an antiviral, an anti-inflammatory or a local anesthetic;
and wherein the eutectic mixture comprises triclosan.

2. The suture thread as claimed in claim 1:
wherein the at least one polymer is at least one biocompatible polymer;
wherein, optionally, the at least one biocompatible polymer is at least one hot melt extrudable biocompatible polymer.

3. The suture thread as claimed in claim 1, wherein the local anesthetic comprises functional groups with strong group electronegativity that is an amide selected from lidocaine, prilocaine, and bupivacaine or an ester selected from procaine, benzocaine and tetracaine, optionally wherein the local anesthetic is lidocaine.

4. The suture thread as claimed in claim 1, wherein the antibacterial, the antifungal, the antiviral, or the anti-inflammatory is selected from antibiotics, non-antibiotic antimicrobials, antivirals, antifungals and antiparasitics comprising an imidazole or nitroimidazole.

5. The suture thread as claimed in any of the preceding claims wherein the polymer is selected from the group comprising polycaprolactone , polyglycolic acid (PGA), polylactic co-glycolic acid (PLGA), polylactic acid (PLA), polydioxanone, poliglecaprone (copolymer of glycolide and epsilon-caprolactone), poly-(trimethylene carbonate)-based polymers, polypropylene, polyester, and nylon.

6. The suture thread as claimed in any of the preceding claims wherein the polymer is biodegradeable or substantially non-biodegradeable.

7. The suture thread as claimed in any of claims 1 to 6 wherein the eutectic mixture comprises triclosan and an anti-inflammatory, wherein the anti-inflammatory is a non-steroidal anti-inflammatory (NSAID), optionally wherein the NSAID comprises a carboxylic acid functionality, optionally wherein the NSAID comprising a carboxylic acid functionality is selected from the group consisting of ibuprofen, naproxen, indomethacin, diclofenac, mefenamic acid, meclofenamic acid, optionally wherein the NSAID is ketoprofen.

8. The suture thread as claimed in any of the preceding claims wherein the two or more eutectic forming components generate eutectic oils in situ within a polymer device.

9. The suture thread as claimed in any of claims 1 to 6 wherein the eutectic mixture comprises at least one fatty acid, optionally wherein the at least one fatty acid is a mono or dicarboxylic, saturated or unsaturated, fatty acid comprising 4 to 20 carbon atoms, optionally wherein the at least one fatty acid is selected from the group consisting of maleic acid , hexanoic acid, hexanedioic acid, capric acid, decanedioic acid , lauric acid (C12), dodecanedioic acid, tetradecanedioic acid, myristic acid, and oleic acid.

10. The suture thread as claimed in any of claims 1 to 6 where the eutectic mixture comprises an antibiotic, optionally wherein the antibiotic is metronidazole.

11. The suture thread as claimed in any of claims 1 to 6 wherein the eutectic mixture comprises triclosan and lidocaine.

12. The suture thread as claimed in any of claims 1 to 7 wherein the eutectic mixture comprises triclosan and ibuprofen or ketoprofen.

13. The suture thread as claimed in any of claims 1 to 6 wherein the eutectic mixture comprises a fatty alcohol, optionally wherein the fatty alcohol is a straight-chain or branched-chain, saturated or unsaturated primary alcohol, ranging from 4-6 carbons to as 26-30 carbons, optionally wherein the fatty alcohol is selected from 3-methyl-3-pentanol ; 1-Dodecanol, Heneicosanol, Elaidyl alcohol, Petroselinyl alcohol, 1-tetradecanol, 1-Docosanol, 1-Tridecanol, 1-Nonadecanol, 1-Triacontanol, 1-Pentadecanol, 1-Nonanol, 1-Tricosanol, Cetyl alcohol, Stearyl alcohol, 1-Decanol, or Isooctyl alcohol.

14. The suture thread as claimed in any of claims 1 to 6 wherein the eutectic mixture comprises a fatty amine, optionally wherein the fatty amine is a straight-chain or branched-chain, saturated or unsaturated amine, optionally wherein the fatty amine is selected from a mono- or di-substituted amine that is optionally substituted with a short chain alkyl at the amino group, optionally wherein the fatty amine comprises from 4-6 carbons to 22-26 carbons, optionally wherein the fatty amine is selected from Pentadecylamine, Hexadecylamine, Docecylamine, Decylamine, Tridecylamine, Octadecylamine, Undecylamine, N, N-dimethyltetradecylamine, Dodecylamine, or Octadecylamine.

15. A method for the production of sutures of any of claims 1 to 14 with drug loadings, optionally wherein the method comprises the use of therapeutic deep eutectic solvent (THEDES) technology, optionally wherein two or more eutectic forming components selected from an acid, a fatty acid, a fatty amine, a fatty alcohol, or any combination of two or more thereof that form, *in situ,* at least one eutectic mixture are co-extruded with at least one polymer at a temperature above the melting point of the polymer.
